# EUROPEAN PATENT APPLICATION

(11) **EP 3 848 032 A1**
(43) Date of publication of application: **14.07.2021**
(21) Application number: 19857755.3
(22) Date of filing: 03.09.2019
(51) Int. Cl.: A61K 31/4745, A61P 43/00

(54) **APPLICATION OF PYRROLOQUINOLINE QUINONE IN PREPARATION OF MEDICAMENT USED FOR PREVENTING AND TREATING ACUTE ALTITUDE SICKNESS AND ACUTE ALTITUDE HYPOXIA INJURY**

(30) Priority: 03.09.2018 CN 201811022046
(71) Applicant: ZheJiang Medicine Co., Ltd. Xinchang Pharmaceutical Factory, Shaoxing, Zhejiang 312500 (CN)
(72) Inventor: SHAO, Dong, Shaoxing, Zhejiang 312500 (CN); SUN, Xinqiang, Shaoxing, Zhejiang 312500 (CN); LAO, Xuejun, Shaoxing, Zhejiang 312500 (CN); LIU, Wei, Shaoxing, Zhejiang 312500 (CN); ZHANG, Weicai, Shaoxing, Zhejiang 312500 (CN)
(74) Representative: Stuttard, Garry Philip
(86) International application number: PCT/CN2019/104174
(87) International publication number: WO 2020/048446

(57) **Abstract**

The present invention relates to an application of pyrroloquinoline quinone (PQQ) in the preparation of a medicament used for preventing and treating acute altitude sickness and acute altitude hypoxia injury. Pyrroloquinoline quinone has the effect of preventing and treating acute high altitude hypoxia injury, and as a drug for the prevention and treatment of acute altitude sickness, the efficacy thereof is equivalent to that of acetazolamide, however acetazolamide has many toxic side effects; meanwhile, as a coenzyme, pyrroloquinoline quinone has the advantages of low toxicity and is easily acceptance by patients. In addition, by means of exhaustive swimming experiments of mice under the conditions of hypoxic exposure, PQQ has been shown to have the feature characteristics of improving the working capabilities of a subject at a high altitude, however acetazolamide has not been found to have said effect.

## Description

### FIELD OF THE INVENTION

The present invention relates to a new application of pyrroloquinoline quinine, specifically, relates to an application of pyrroloquinoline quinone (PQQ) in preparation of medicament for preventing and treating acute altitude hypoxia injury.

### BACKGROUND OF THE INVENTION

Low pressure and low oxygen in plateau areas are main environmental factors in plateau areas and main pathogenic factors of altitude sickness. With the development of the western part of the country and the opening of the Qinghai-Tibet Railway, more and more people work and travel on the plateau is increasing. People pay more and more attention to health maintenance of people entering the plateau area. It has become an important direction of plateau medicine or health care product research.

Currently, treatment for high altitude polycythemia requires more than 250mg of acetazolamide daily. However, acetazolamide can easily cause adverse reactions such as perchloric metabolic acidosis, limb numbness, gastrointestinal discomfort, confusion of consciousness, nausea, anorexia, drowsiness, polyuria and tinnitus. The tissue/organ distribution, subcellular localization and physiological function of them are quite different, because carbonic anhydrase has 12 isoenzymes with enzymatic catalytic activity. Acetazolamide has strong inhibitory effects on a variety of carbonic anhydrase isozymes. Research on enzyme inhibitors will mainly focus on finding carbonic anhydrase inhibitors with strong selectivity and high tissue specificity.

Pyrroloquinoline quinone (PQQ) is a coenzyme different from pyridine nucleotides (NAD, NADP) and riboflavin (FMN, FAD). It is an oxidoreductase prosthetic group that acts as an electron donor and receptor participates in the electron transfer of the respiratory chain in the redox process, has strong free radical scavenging ability, and has many physiological functions. It plays an important role in the process of stimulating metabolism of organisms, promoting growth and development, protecting liver injury, degrading ethanol, anti-oxidation and anti-radiation. However, there is no research and application report on uses of pyrroloquinoline quinone for preventing and treating acute altitude hypoxia injury.

### SUMMARY OF THE INVENTION

In order to overcome the above shortcomings, the present invention provides an application of pyrroloquinoline quinone (PQQ) in the preparation of a medicament for preventing and treating acute altitude sickness and acute altitude hypoxia injury.

Pyrroloquinoline quinone of the present invention has effects of preventing and treating acute altitude hypoxia injury. Its efficacy of pyrroloquinoline quinone is equivalent to that of acetazolamide in a medicine for preventing and treating acute altitude sickness. But acetazolamide has serious side effects. Pyrroloquinoline quinone as a coenzyme has advantages of low toxicity and easy acceptance by patients. In addition, PQQ shows through exhaustive swimming experiments in mice under low oxygen exposure conditions that pyrroloquinoline quinone has function characteristics of improving the working ability of the plateau bodies, but no effect of Acetazolamide is found.

### DETAILED DESCRIPTION AND PREFERRED EMBODIMENTS THEREOF

The following examples are used to further specifically illustrate the present invention, but not limited to the following examples and the range of process parameters in the examples.

### 1. Material

### 1.1 Experimental animals

Kunming mice, clean grade, female/male, weight 16-22g, provided by the Animal Center of the Academy of Military Medical Sciences.

Wistar rat, clean grade, male, weight 160-220g, provided by the Animal Center of the Academy of Military Medical Sciences.

### 1.2 Main reagents

PQQ (XINCHANG PHARMACEUTICAL FACTORY, batch number: 150502)
Acetazolamide, content: 99.9%, (Chengdu Youlian Biotechnology Co., Ltd., batch number: 260905091)
NO kit, Coomassie brilliant blue kit (Beijing Puerweiye Biotechnology Co., Ltd.)
ET-1 Enzyme-linked Immunoassay (ELISA) Kit (Beijing Puer Weiye Biotechnology Co., Ltd.)
Oxidative stress SOD, CO, MDA detection kit (Nanjing Jiancheng Technology Co., Ltd.)
Test kit for adenosine triphosphate, lactic acid, hepatic glycogen detection kit (Nanjing Jiancheng Technology Co., Ltd.)
Pentobarbital sodium (American Sigma company).

### 1.3 Main instruments

Multi-factor compound environment simulation medical science experimental chamber (AVIC Guizhou Fenglei Aviation Ordnance Co., Ltd.)
FlexStation 3 multi-functional enzyme label instrument workstation (US Molecular Devices Company)
7180 Automatic blood biochemical analyzer (Hitachi, Japan)
Heraeus low-temperature high-speed centrifuge (Germany Kendro Company)
Electronic balance (Germany Sartorius Company)
Ultra-low temperature refrigerator (Japan Sanyo Corporation)

### 2. PQQ anti-acute high altitude hypoxia injury experiment

### 2.1 Rat acute decompression hypoxia experiment

Select healthy Wistar rats weight 160-200g, half female and half male. After feeding for 3 days, these Wistar rats are randomly divided into ten groups according to their body weight, each group has 16±2 rats. The groups are divided as follows: five groups of the hypoxic exposure drug test group, respectively, are given 0.91 mg/kg, 1.83 mg/kg, 3.66 mg/kg, 7.31 mg/kg, and 14.63 mg/kg drugs. One group of hypoxia exposure positive acetazolamide control group is given 0.11g/kg acetazolamide; one group of hypoxia model group and one group of normal oxygen control group, given a corresponding volume of solvent. In addition, set up one group of normal oxgen PQQ control group (given PQQ 3.66 mg/kg) and one group of normal oxygen acetazolamide control group (given 0.11 g/kg acetazolamide). After continuous intragastric administration for 7 days, the animals in the hypoxic exposure group are simultaneously put into the decompression and hypoxia compound experimental chamber, the hatch is closed, and the pressure is reduced at a speed of 10m/s. After being raised to an altitude of 6000m and maintained for 8 hours, the speed in 10m/s drops is reduced to normal altitude, open the hatch, and then take out the animal, collect blood samples (after standing for 1 hour, the supernatant is centrifuged and stored at -20°C), and liver tissue samples. Detection indicator includes body weight, serum blood glucose, ATP, lactic acid, endothelin, nitric oxide, SOD, CO, MDA related to oxidative stress, and total protein, albumin, triglycerides, total cholesterol, high-density lipoprotein cholesterol, low-density lipoprotein cholesterol, glutamic pyruvic transaminase, total bilirubin, gluamic oxalacetic transaminase, serum urea nitrogen, creatinine, uric acid, lactate dehydrogenase, creatine kinase, α-hydroxybutyrate dehydrogenase; liver ATP, hepatic glycogen. Except that the formal oxygen control animal group is not put into the decompression and hypoxia compound experimental chamber, other experimental conditions and detection indicators are the same.

### 2.2 Exhaustive swimming experiment in mice exposed to high altitude hypoxia

Select healthy male Kunming mice weight 18-22g. After feeding for 3 days, they are randomly divided into seven groups according to their body weight, each group has 10±1 mice. The groups are divided as follows: 5 groups of the drug test group, respectively, are given 1.32 mg/kg, 2.64 mg/kg, 5.28 mg/kg, 10.56 mg/kg, and 21.12 mg/kg drugs by gavage administration. One positive drug control group of the acetazolamide is given 0.16g/kg acetazolamide; one negative control group is given a corresponding volume of solvent. After administration for 7 days, select an exhaustive swimming experiment under hypoxic conditions perform physical work ability evaluation; the observation index is an exhaustive swimming time. In the decompression and hypoxia compound laboratory chamber, decompression hypoxia is performed to an altitude of 6000m, the swimming box is filled with water at a depth of 40cm and the water temperature is 25°C. The animal is placed in the swimming box, and use a stopwatch to record a time from the start of swimming to exhaustion of the animal. Exhaustive swimming time is the time when an animal still cannot surface for 9s after sinking.

### 3. Detection method

### 3.1 Determination of nitric oxide content in serum

Nitric oxide has very reactive chemically, and its metabolism in the body quickly turns to NO²⁻ and NO³⁻, and NO²⁻ further turns to NO³⁻. This method uses nitrate reductase specificity reduce NO³⁻ to NO²⁻, and then determine the level of its concentration by displaying shades of color. Operate according to the kit instructions, measure the absorbance of each tube at a wavelength of 550nm, and then calculate the content of nitric oxide of the sample to be tested according to the formula.

### 3.2 Determination of serum endothelin-1 content

To the coated microwells pre-coated with rat endothelin (endothelin-1) capture antibody, add in sequence specimens, standards, and HRP-labeled detection antibody, and then incubate and wash thoroughly. Use a substrate TMB for color development. TMB is converted into blue under the catalysis of peroxidase and converted into the final yellow color under the action of acid. The color intensity is positively correlated with the rat endothelin (endothelin-1) in the sample. Measure the absorbance (OD value) by a microplate reader at a wavelength of 450nm, take the concentration of the standard as the abscissa, and take the corresponding OD value as the ordinate, draw a linear regression curve of the standard, and calculate the concentration of each sample according to the curve equation.

### 3.3 Determination of oxidative stress indicators such as malondialdehyde, superoxide dismutase, total antioxidant capacity

**Determination of malondialdehyde:** The malondialdehyde in the degradation product of lipid peroxide is condensed with thiobarbital (TBA) to form a red product with a maximum absorption peak at 532nm. Operate according to the instructions, and then measure the absorbance value of each tube, and afterwards calculate the content of malondialdehyde in the sample to be tested according to the formula.

**Determination of superoxide dismutase:** Superoxide anion free radicals (O²⁻) are generated through the reaction system of xanthine and xanthine oxidase. The latter oxidizes hydroxylamine to form nitrite. It appears purplish red under the action of chromogenic agent. Measure its absorbance by a visible light spectrophotometer. When the measured sample contains superoxide dismutase, it has specific inhibitory effect on superoxide anion free radicals, make formed nitrite reduced. The absorbance value of the measuring tube is lower than that of the control tube during colorimetry. The superoxide dismutase activity in the tested sample can be calculated by formula calculation. Operate according to the instructions, measure the absorbance value of each tube at a wavelength of 550nm, and then calculate the activity of superoxide dismutase in the sample to be tested according to the formula.

**Determination of total antioxidant capacity (T-AOC):** Fe³⁺ can be reduced to Fe²⁺ by using antioxidant substances in the body. The latter can form a stable complex with phenanthrophins and measure its antioxidant capacity by colorimetry. Operate according to the kit instructions, measure the absorbance of each tube at a wavelength of 520nm, and then calculate the total antioxidant capacity in the sample to be tested according to the formula.

### 3.4 Determination of adenosine triphosphate, lactic acid and liver glycogen

**Determination of adenosine triphosphate (ATP):** Perform enzyme-linked immunosorbent assay by double-antibody one-step sandwich method. To the coated microwells pre-coated with rat adenosine triphosphate (ATP) capture antibody, add in sequence the specimens, standard, and HRP-labeled detection antibody, and then incubate and wash thoroughly. Use a substrate TMB for color development, TMB is converted into blue under the catalysis of peroxidase and converted into the final yellow color under the action of acid. The color intensity is positively correlated with the rat adenosine triphosphate (ATP) in the sample. Measure the absorbance (OD value) by a microplate reader at a wavelength of 450 nm, take the concentration of the standard as the abscissa, and take the corresponding OD value as the ordinate, draw a linear regression curve of the standard, and calculate the concentration of each sample according to the curve equation.

**Determination of lactic acid (LC):** Perform enzyme-linked immunosorbent assay by double-antibody one-step sandwich method. To the coated microwells pre-coated with rat lactic acid (LC) capture antibody, add in sequence the specimens, standard, and HRP-labeled detection antibody, and then incubate and wash thoroughly. Use a substrate TMB for color development. TMB is converted into blue under the catalysis of peroxidase and converted into the final yellow color under the action of acid. The color intensity is positively correlated with the rat lactic acid (LC) in the sample. Measure the absorbance (OD value) by a microplate reader at a wavelength of 450 nm, take the concentration of the standard as the abscissa, and take the corresponding OD value as the ordinate, draw a linear regression curve of the standard, and then calculate the concentration of each sample according to the curve equation.

**Determination of liver glycogen (GC):** Perform enzyme-linked immunosorbent assay by double-antibody one-step sandwich method. To the coated microwells pre-coated with rat liver glycogen (GC) capture antibody, add in sequence the specimens, standard, and HRP-labeled detection antibody, and then incubate and wash thoroughly. Use a substrate TMB for color development, TMB is converted into blue under the catalysis of peroxidase and converted into the final yellow color under the action of acid. The color intensity is positively correlated with the rat liver glycogen (GC) in the sample. Measure the absorbance (OD value) by a microplate reader at a wavelength of 450 nm, take the concentration of the standard as the abscissa, and take the corresponding OD value as the ordinate, draw a linear regression curve of the standard, and calculate the concentration of each sample according to the curve equation.

### 3.5 Determination of blood biochemical indexes and blood homocysteine

Measure blood glucose, total protein, albumin, triglycerides, total cholesterol, high-density lipoprotein cholesterol, low-density lipoprotein cholesterol, glutamic-pyruvic transaminase, total bilirubin, glutamic oxalacetic transaminase, serum urea nitrogen, creatinine, uric acid, lactate dehydrogenase, creatine kinase, α-hydroxybutyrate dehydrogenase, blood homocysteine by using an automatic blood biochemical analyzer.

### 4. Statistical analysis

The measurement data is processed by t-test and variance analysis, and the experimental results are expressed as mean ± standard error (x ± s); count data are expressed by using x² test and direct probability method P<0.05 to indicate that the difference has significant.

### 5. Experimental results of PQQ's anti-acute altitude hypoxia injury in mice

### 5.1 Hypoxia tolerance test of simulated 10000m altitude acute decompression in mice

### 5.1.1 Single dose test

**Table 1 Mice weight before hypoxia tolerance test of simulated 10000m altitude acute decompression in mice (single dose test)**

| Group | Male(g) | Female(g) |
|---|---|---|
| Negative control (equal volume of water) | 19.38±0.53 | 16.90±1.33 |
| Acetazolamide positive drug control group(0.16g/kg) | 19.47±0.57 | 17.31±1.22 |
| PQQ drug dose group I (2.64mg/kg) | 19.71±0.52 | 17.25±1.23 |
| PQQ drug dose group II (5.28mg/kg) | 19.51±0.68 | 17.32±1.35 |
| PQQ drug dose group III(10.56mg/kg) | 19.16±0.34 | 17.24±0.96 |

It can be seen from Table 1 that there is no statistically significant difference in the body weight of male and female mice of each group before the experiment (P>0.05).

### 5.1.2 Administration for three days

**Table 3 Mice weight before hypoxia tolerance test of simulated 10000m altitude acute decompression in mice (administration for three days)**

| Group | Male(g) | Female(g) |
|---|---|---|
| Negative control (equal volume of water) | 19.38±1.41 | 18.12±1.23 |
| Acetazolamide positive drug control group(0.16g/kg) | 18.29±0.85 | 17.80±0.94 |
| PQQ drug dose group I (1.32mg/kg) | 19.97±0.84 | 18.22±1.16 |
| PQQ drug dose group II (2.64mg/kg) | 19.65±0.83 | 18.79±1.14 |
| PQQ drug dose group III(5.28mg/kg) | 20.16±1.18 | 18.10±0.76 |
| PQQ drug dose group IV(10.56mg/kg) | 19.73±0.85 | 17.95±1.14 |
| PQQ drug dose group V (21.12mg/kg) | 19.28±0.95 | 18.48±1.01 |

It can be seen from Table 3 that there is no statistically significant difference in body weight of the male and female mice of each group before the experiment (P>0.05).

### 5.1.3 Administration for seven days

**Table 5 Changes of mice weight before hypoxia tolerance test of simulated 10000m altitude acute decompression in mice (administration for seven days)**

| Group | Male(g) | | Female(g) | |
|---|---|---|---|---|
| | 1 day | 7 days | 1 day | 7 days |
| Negative control (equal volume of water) | 16.62±2.64 | 22.11±4.05 | 17.89±1.44 | 22.48±1.94 |
| Acetazolamide positive drug control group(0.16g/kg) | 17.31±1.58 | 21.43±2.79 | 17.58±1.93 | 19.69±3.52 |
| PQQ drug dose group I (1.32mg/kg) | 18.46±1.36 | 22.19±2.69 | 18.10±1.19 | 21.36±1.98 |
| PQQ drug dose group II (2.64mg/kg) | 18.31±1.01 | 23.51±3.09 | 18.19±1.24 | 21.40±1.92 |
| PQQ drug dose group III (5.28mg/kg) | 18.38±1.85 | 22.04±3.25 | 17.95±1.28 | 22.71±1.38 |
| PQQ drug dose group IV(10.56mg/kg) | 18.26±1.15 | 22.41±1.79 | 17.99±1.41 | 22.66±1.68 |
| PQQ drug dose group V (21.12mg/kg) | 18.00±1.78 | 23.54±3.92 | 18.35±1.62 | 23.94±2.10 |

It can be seen from Table 5 that there is no statistically significant difference in body weight of the male and female mice of each group before the experiment (P>0.05)., There is no statistically significant difference in body weight of male and female mice of each group after administration for 7 days (P>0.05).

**Table 7 Changes of male mice weight before hypoxia tolerance test of simulated 10000m altitude acute decompression in mice (administration for seven days) (repeat the experiment in male mice after administration for seven days)**

| Group | Male(g) | |
|---|---|---|
| | 1 day | 7 days |
| Negative control (equal volume of water) | 19.36±1.24 | 25.79±4.00 |
| Acetazolamide positive drug control group (0. 16g/kg) | 19.53±0.93 | 21.35±2.47** |
| PQQ drug dose group I (1.32mg/kg) | 19.84±1.84 | 27.14±2.66 |
| PQQ drug dose group II (2.64mg/kg) | 19.66±1.08 | 26.78±3.14 |
| PQQ drug dose group III(5.28mg/kg) | 19.75±1.27 | 26.47±2.88 |
| PQQ drug dose group IV(10.56mg/kg) | 19.90±1.06 | 27.33±2.27 |
| PQQ drug dose group V (21.12mg/kg) | 19.85±1.00 | 26.73±2.37 |

It can be seen from Table 7 that there is no statistically significant difference in the body weight of the male mice of each group before the experiment (P>0.05). After administration for 7 days, compared with the control group, the mice weight in the acetazolamide-positive drug control group is decreased (P<0.05), and but there is no statistically significant difference in body weight in each PQQ drug dose group (P>0.05).

### 5.1.4 14 Days of administration

**Table 9 Changes of mice weight before hypoxia tolerance test of simulated 10000m altitude acute decompression in mice (administration for 14 days)**

| Group | Male(g) | | | Female(g) | |
|---|---|---|---|---|---|
| | 1 day | 7 days | 14 days | 1 day | 7 days |
| Negative control (equal volume of water) | 16.33±1.66 | 22.61±1.83 | 28.82±2.77 | 15.82±1.72 | 21.87±2.03 |
| Acetazolamide positive drug control group(0.16g/kg) | 14.66±1.52* | 16.29±3.25** | 21.16±3.61** | 15.24±0.89 | 19.35±3.12* |
| PQQ drug dose group I (1.32mg/kg) | 16.36±1.47 | 22.88±1.78 | 30.85±2.32 | 16.13±1.20 | 20.45±1.37 |
| PQQ drug dose group II (2.64mg/kg) | 16.63±1.87 | 22.58±1.41 | 28.13±2.70 | 15.75±1.07 | 21.41±1.62 |
| PQQ drug dose group III (5.28mg/kg) | 17.01±1.91 | 23.68±2.02 | 29.44±2.15 | 15.63±1.12 | 20.47±2.11 |
| PQQ drug dose group IV(10.56mg/kg) | 16.58±1.68 | 23.13±1.73 | 19.58±2.11 | 15.84±1.33 | 21.68± 1.66 |
| PQQ drug dose group V (21.12mg/kg) | 16.86±1.71 | 23.67±1.77 | 30.81±2.76 | 15.68±0.91 | 20.10±2.07 |

| | | | | | |
|---|---|---|---|---|---|
| *p<0.05, **p<0.01 VS negative control group | | | | | |

It can be seen from Table 9 that compared with the negative control group, there is no statistically significant difference in the mice weight of the male and female of each group before administration (P>0.05). After administration for 7 days, the male and female acetazolamide positive drug control groups had lower body weights than their corresponding negative control groups, and has a statistically significant difference (P< 0.05). Compared with its negative control group, the body weight of each PQQ drug dose group has no statistically significant difference (P>0.05). After administration for 14 days, the body weight of the male and female acetazolamide-positive drug control groups is lower than that of the negative control group, and has a statistically significant difference (P<0.05). Compared with the female negative control group, the body weight of female mice PQQ drug-dose groups III and V is decreased, and has a statistically significant difference (P<0.05). Compared with their corresponding negative control group, The body weight of female and male mice in other PQQ drug dose group has no statistically significant difference (P>0.05).

### 6. Experimental results of closed hypoxia tolerance in mice

### 6.1 Test of closed hypoxia tolerance in male mice

**Table 11 Changes of body weight of male mice before closed hypoxia tolerance test**

| Group | weight (g) | |
|---|---|---|
| | 1 day | 3 days |
| Negative control (equal volume of water) | 15.43±1.14 | 18.12±1.65 |
| Acetazolamide positive drug control group(0.16g/kg) | 15.41±1.08 | 15.88±1.24** |
| PQQ drug dose group I (1.32mg/kg) | 15.46±1.04 | 17.74±1.30 |
| PQQ drug dose group II (2.64mg/kg) | 15.40±1.03 | 17.46±1.29 |
| PQQ drug dose group III(5.28mg/kg) | 15.51±1.11 | 17.41±1.23 |
| PQQ drug dose group IV(10.56mg/kg) | 15.32±1.10 | 16.20±1.03 |
| PQQ drug dose group V (21.12mg/kg) | 15.45±0.97 | 17.54±1.14 |

| | | |
|---|---|---|
| *p<0.05, **p<0.01 VS negative control group | | |

It can be seen from Table 11 that there is no statistically significant difference in the body weight of the male mice of each group before the experiment (P>0.05). After administration for 3 days, compared with the control group, the mice in the acetazolamide-positive control group is decreased (P<0.05), and has no statistically significant difference in body weight of the PQQ drug dosage groups (P>0.05).

### 6.2 Confined hypoxia tolerance test in famale mice

**Table 14 Changes of body weight of famale mice before closed hypoxia tolerance test**

| Group | weight (g) | |
|---|---|---|
| | 1 day | 3 days |
| Negative control (equal volume of water) | 20.81±0.70 | 22.33±0.72 |
| Acetazolamide positive drug control group(0.16g/kg) | 20.27±0.90 | 20.88±1.50* |
| PQQ drug dose group I (1.32mg/kg) | 20.31±1.29 | 22.33±1.91 |
| PQQ drug dose group II (2.64mg/kg) | 19.83±1.19* | 22.22±1.28 |
| PQQ drug dose group III(5.28mg/kg) | 20.58±1.07 | 22.52±1.71 |
| PQQ drug dose group IV(10.56mg/kg) | 20.13±1.64 | 21.92±1.83 |
| PQQ drug dose group V (21.12mg/kg) | 20.19±1.34 | 22.49±1.77 |

| | | |
|---|---|---|
| *p<0.05, **p<0.01 VS negative control group | | |

It can be seen from Table 14 that there is no statistically significant difference in the body weight of the female mice of each group before the experiment (P>0.05). After administration for 3 days, compared with the control group, the mice weight in the acetazolamide-positive control group is decreased (P<0.05), and has no statistically significant difference in body weight of the PQQ drug dosage groups (P>0.05).

### 7. Experimental results of PQQ anti-acute high altitude hypoxia injury in rats

### 7.1 Changes of rat body weight before the acute decompression hypoxia experiment

**Table 17 Changes of rat body weight before the acute decompression hypoxia experiment**

| Group | Male(g) | | Female(g) | |
|---|---|---|---|---|
| | 1 day | 7 days | 1 day | 7 days |
| Normoxia control group (equal volume of water) | 172.3±7.7 | 200.1±7.8 | 152.7±8.6 | 171.9±9.1 |
| Hypoxia model group(equal volume of water) | 171.6±6.3 | 200.8±10.8 | 153.4±9.2 | 169.2±7.0 |
| PQQ drug dose group I (0.91mg/kg) | 173.4±7.4 | 196.4±5.7 | 154.4±7.2 | 169.6±3.3 |
| PQQ drug dose group II (1.83mg/kg) | 173.6±5.9 | 206.8±6.2 | 153.6±6.9 | 167.4±6.7 |
| PQQ drug dose group III (3.66mg/kg) | 170.8±10.8 | 198.3±9.0 | 154.1±8.6 | 172.1±2.2 |
| PQQ drug dose group IV (7.31 mg/kg) | 169.9±9.9 | 198.6±11.6 | 154.3±7.7 | 171.1±8.6 |
| PQQ drug dose group V (14.63mg/kg) | 171.6±10 | 199.6±12.5 | 152.7±7.2 | 166.0±4.1 |
| Acetazolamide positive drug control group(0.11g/kg) | 172.4±6.8 | 189.1±11.8*# | 153.4±6.1 | 163.3±6.76 |

| | | | | |
|---|---|---|---|---|
| *p<0.05, **p<0.01 VS normoxia control group, #p<0.05, ##p<0.01 VS hypoxia model group | | | | |

It can be seen from Table 17 that compared with the normoxia control group and the hypoxic model group, the body weight of the acetazolamide-positive drug control group of male rats is decreased after intragastric administration for 7 days, has a statistically significant difference (P< 0.05). Other groups has no statistically significant difference (P>0.05). Compared with the normoxia control group and the hypoxic model group, there is no statistically significant difference in the body weight of female rats before gavage and after gavage for 7 days (P>0.05).

### 7.2 Effects of PQQ on blood glucose in rats exposed to acute altitude hypoxia

**Table 18 Effects of PQQ on blood glucose in male rats exposed to acute altitude hypoxia**

| Group | Blood sugar(mmol/L) |
|---|---|
| Normoxia control group (equal volume of water) | 8.53±1.54 |
| Hypoxia model group(equal volume of water) | 8.66±1.78 |
| PQQ drug dose group I (0.91mg/kg) | 7.35±1.94 |
| PQQ drug dose group II (1.83mg/kg) | 8.21±1.46 |
| PQQ drug dose group III (3.66mg/kg) | 8.97±1.44 |
| PQQ drug dose group IV(7.31mg/kg) | 8.28±1.57 |
| PQQ drug dose group V (14.63mg/kg) | 8.85±2.04 |
| Acetazolamide positive drug control group(0.11g/kg) | 8.87±1.24 |

| | |
|---|---|
| *p<0.05, **p<0.01 VS normoxia control group, #p<0.05, ##p<0.01 VS hypoxia model group | |

It can be seen from Table 18 that compared with the normoxia control group and the hypoxia model group, there is no statistically significant difference in blood glucose values of the hypoxia model group and the PQQ each drug dosage group (P>0.05).

**Table 19 Effects of PQQ on blood glucose in female rats exposed to acute altitude hypoxia**

| Group | **B**lood sugar(mmol/L) |
|---|---|
| Normoxia control group (equal volume of water) | 8.39±0.44 |
| Hypoxia model group(equal volume of water) | 6.87±0.54** |
| PQQ drug dose group I (0.91mg/kg) | 8.59±0.48## |
| PQQ drug dose group II (1.83mg/kg) | 8.28±0.84## |
| PQQ drug dose group III (3.66mg/kg) | 8.27±0.37## |
| PQQ drug dose group IV(7.31mg/kg) | 7.91±0.65## |
| PQQ drug dose group V (14.63mg/kg) | 7.55±0.48**# |
| Acetazolamide positive drug control group(0.11g/kg) | 7.63±0.76*# |
| Normoxia PQQ drug group(3.66mg/kg) | 8.13±0.57 |
| Normoxia acetazolamide group(0.11g/kg) | 7.18±0.39** |

| | |
|---|---|
| *p<0.05, **p<0.01 VS normoxia control group, #p<0.05, ##p<0.01 VS hypoxia model group | |

It can be seen from Table 19 that compared with the normoxia control group, the blood glucose values of female rats in the hypoxia model group, the PQQ drug dose group V after hypoxia exposure, the acetazolamide positive drug control group and the normoxia acetazolamide group are decreased and have a statistically significant difference (P<0.05). And other groups have no statistically significant difference compared with the normoxia control group (P>0.05). Compared with the hypoxia model group, the blood glucose values of female rats in the PQQ drug dose groups and the acetazolamide-positive drug control group are increased, and have statistically significant differences (P<0.05).

**7.3 Effects of PQQ on protein metabolism in rats exposed to acute altitude hypoxia Table 20 Effects of PQQ on protein metabolism in male rats exposed to acute altitude hypoxia**

| Group | albumin (g/L) | albumin(g/L) |
|---|---|---|
| Normoxia control group (equal volume of water) | 51.05±2.50 | 26.34±0.98 |
| Hypoxia model group(equal volume of water) | 52.42±1.47 | 28.31±1.08** |
| PQQ drug dose group I (0.91mg/kg) | 42.59±4.24**## | 22.55±2.53**## |
| PQQ drug dose group II (1.83mg/kg) | 49.30±3.35# | 26.14±2.04# |
| PQQ drug dose group III (3.66mg/kg) | 52.71±1.20 | 28.60±1.12** |
| PQQ drug dose group IV(7.31mg/kg) | 52.55±2.78 | 28.06±1.31** |
| PQQ drug dose group V (14.63mg/kg) | 52.97±1.38 | 28.38±0.90** |
| Acetazolamide positive drug control group(0.11g/kg) | 53.14±2.18 | 27.87±1.140* |

| | | |
|---|---|---|
| *p<0.05, **p<0.01 VS normoxia control group, #p<0.05, ##p<0.01 VS hypoxia model group | | |

It can be seen from Table 20 that compared with the normoxia control group, the serum total protein value of male rats for the hypoxia model group and the PQQ drug dose groups (except PQQ drug dose group I) has no statistically significant difference (P>0.05). Compared with the normoxia control group, the serum total protein value of PQQ drug dose group I is decreased, and has a statistically significant difference (P<0.05). Compared with the hypoxia model group, the total serum protein value of PQQ drug dose group I and PQQ drug dose group II is decreased, and has a statistically significant decrease (P<0.05). There is no statistically significant difference in other PQQ drug dose groups (P>0.05).

Compared with the normoxia control group, the serum albumin value of male rats for the hypoxia model group and PQQ drug dose group I , III, IV, V is increased (P<0.05). Compared with the normoxia control group, PQQ drug dose group II has no statistically significant difference (P>0.05). Compared with the hypoxia model group, the serum albumin values of the PQQ drug dose group I and the PQQ drug dose group II are decreased, and have a statistically significant difference (P<0.05). Other PQQ drug dose groups have no statistically significant difference (P>0.05).

**Table 21 Effects of PQQ on protein metabolism in famale rats exposed to acute altitude hypoxia**

| Group | albumin (g/L) | albumin(g/L) |
|---|---|---|
| Normoxia control group (equal volume of water) | 55.32±2.00 | 30.28±1.05 |
| Hypoxia model group(equal volume of water) | 55.43±1.77 | 31.40±1.11* |
| PQQ drug dose group I (0.91mg/kg) | 54.56±1.24 | 31.03±0.98 |
| PQQ drug dose group II (1.83mg/kg) | 53.04±1.71*# | 29.67±0.98## |
| PQQ drug dose group III (3.66mg/kg) | 52.44±1.12**## | 29.33±0.62## |
| PQQ drug dose group IV(7.31mg/kg) | 55.67±1.23 | 31.49±0.70* |
| PQQ drug dose group V (14.63mg/kg) | 53.91±2.09 | 30.16±1.40 |
| Acetazolamide positive drug control group(0.11g/kg) | 53.90±2.23 | 29.18±1.68# |
| Normoxia PQQ drug group(3.66mg/kg) | 56.53±1.50 | 30.87±1.41 |
| Normoxia acetazolamide group(0.11g/kg) | 59.00±1.59* | 32.13±0.45* |

| | | |
|---|---|---|
| *p<0.05, **p<0.01 VS normoxia control group, #p<0.05, ##p<0.01 VS hypoxia model group | | |

It can be seen from Table 21 that compared with the normoxia control group, the total serum protein values of female rats in the PQQ drug dose group II and III are decreased (P<0.05), and the normoxia acetazolamide group has a statistically significant increase (P <0.05), there is no statistically significant difference in other groups (P>0.05). Compared with the hypoxia model group, the total serum protein values of the PQQ drug dose group II and III are decreased (P<0.05), but there is no statistically significant difference in the other groups (P>0.05).

Compared with the normoxia control group, the serum albumin values of female rats in the hypoxia model group, the PQQ drug dose group IV after hypoxia exposure, and the normoxia acetazolamide group are increased (P<0.05), and there is no statistically significant difference in the other groups (P>0.05). Compared with the hypoxia model group, the serum albumin values of the PQQ drug dose group II, the PQQ drug dose group III and the acetazolamide positive drug control group are decreased (P<0.05), and there is no statistically significant difference in the other groups (P>0.05).

**7.4 Effects of PQQ on lipid metabolism in rats exposed to acute altitude hypoxia Table 22 Effects of PQQ on lipid metabolism in male rats exposed to acute altitude hypoxia**

| Group | Triglycerides (mmol/L) | Total cholesterol (mmol/L) | High density lipoprotein cholesterol(mmol/L) | Low density lipoprotein cholesterol(mmol/L) |
|---|---|---|---|---|
| Normoxia control group (equal volume of water) | 1.05±0.29 | 1.92±0.14 | 1.36±0.13 | 0.25±0.03 |
| Hypoxia model group(equal volume of water) | 1.08±0.39 | 1.80±0.14 | 1.33±0.09 | 0.18±0.03* |
| PQQ drug dose group I (0.91mg/kg) | 1.25±0.64 | 1.47±0.15**## | 1.03±0.09**## | 0.16±0.03** |
| PQQ drug dose group II (1.83mg/kg) | 1.06±0.35 | 1.71±0.90** | 1.23±0.11 | 0.20±0.02** |
| PQQ drug dose group III (3.66mg/kg) | 1.36±0.58 | 1.80±0.16 | 1.28±0.09 | 0.19±0.04** |
| PQQ drug dose group IV (7.31 mg/kg) | 1.35±0.36 | 1.69±0.10** | 1.23±0.10*# | 0.16±0.02** |
| PQQ drug dose group V(14.63mg/kg) | 1.40±0.74 | 1.95±0.15# | 1.44±0.15 | 0.19±0.03** |
| Acetazolamide positive drug control group(0.11g/kg) | 1.05±0.49 | 1.88±0.21 | 1.39±0.14 | 0.21±0.05 |

| | | | | |
|---|---|---|---|---|
| *p<0.05, **p<0.01 VS normoxia control group, #p<0.05, ##p<0.01 VS hypoxia model group | | | | |

It can be seen from Table 22 that compared with the normoxia control group and hypoxia model group, the serum triglyceride content of male rats in each drug intervention group has no statistically significant difference (P>0.05).

Compared with the normoxia control group, the total serum cholesterol values of male rats in the PQQ drug dose group I, the PQQ drug dose group II and the PQQ drug dose group IV are decreased (P<0.05), and there is no statistically significant difference in the other groups (P>0.05). Compared with the hypoxia model group, the total serum cholesterol value of male rats in the PQQ drug dose group I is decreased (P<0.05), and the total serum cholesterol value of the PQQ drug dose group V is increased (P<0.05), and there is no statistically significant difference in the other groups (P>0.05).

Compared with the normoxia control group, the serum high-density lipoprotein cholesterol values of male rats in PQQ drug dose group I and the PQQ drug dose group IV are decreased (P<0.05), and there is no statistically significant difference in the other groups (P> 0.05). Compared with the hypoxia model group, the serum HDL cholesterol values of male rats in PQQ drug dose group I and PQQ drug dose group IV are decreased (P<0.05), and there is no statistically significant difference in the other groups (P>0.05) ).

Compared with the normoxia control group, the serum low-density lipoprotein cholesterol values of male rats in each group are reduced, except for the acetazolamide positive drug control group, the other groups have statistically significant differences (P<0.05). Comparing with hypoxia model group, the serum low-density lipoprotein cholesterol values of male rats in each drug treatment group has no statistically significant difference (P>0.05).

**Table 23 the effect of PQQ on lipid metabolism in famale rats exposed to acute altitude hypoxia**

| Group | Total cholesterol (mmol/L) | High density lipoprotein cholesterol(mmol/L) | Low density lipoprotein cholesterol(mmol/L) |
|---|---|---|---|
| Normoxia control group (equal volume of water) | 1.89±0.11 | 1.35±0.07 | 0.16±0.13 |
| Hypoxia model group(equal volume of water) | 1. 67±0.13** | 1.27±0.10 | 0.11±0.02** |
| PQQ drug dose group I(0.9 1mg/kg) | 1.69±0.11** | 1.30±0.07 | 0.12±0.01** |
| PQQ drug dose group II(1.83mg/kg) | 1.56±0.17** | 1.18±0.14** | 0.10±0.02** |
| PQQ drug dose group III(3.66mg/kg) | 1.47±0.11**## | 1.14±0.10**# | 0.10±0.02** |
| PQQ drug dose group IV(7.31mg/kg) | 1.69±0.09** | 1.27±0.09 | 0.10±0.02** |
| PQQ drug dose group V(14.63mg/kg) | 1.64±0.13** | 1.28±0.11 | 0.11±0.02** |
| Acetazolamide positive drug control group(0.11g/kg) | 2.02±0.20## | 1.55±0.19*## | 0.17±0.04## |
| Normoxia PQQ drug group(3.66mg/kg) | 1.87±0.10 | 1.39±0.10 | 0.14±0.03 |
| Normoxia acetazolamide groupe(0.11g/kg) | 2.14±0.10** | 1.67±0.07** | 0.16±0.02 |

| | | | |
|---|---|---|---|
| *p<0.05, **p<0.01 VS normoxia control group, #p<0.05, ##p<0.01 VS hypoxia model group | | | |

It can be seen from Table 23 that compared with the normoxia control group, the total serum cholesterol values of female rats in the hypoxia model group and the PQQ drug dosage groups after hypoxia exposure are decreased (P<0.05), and the acetazolamide positive drug control group after hypoxia exposure has no statistically significant difference, while the serum total cholesterol value of the normoxazinamide group is increased, and there is a statistically significant difference (P<0.05). The acetazolamide positive drug control group and the normoxia PQQ drug group have no statistically significant difference (P>0.05). Compared with the hypoxia model group, the total serum cholesterol value of female rats in the PQQ drug dose group III is decreased (P<0.05), and the total serum cholesterol value of the acetazolamide-positive drug control group is increased (P<0.05), the other groups have no statistically significant difference (P>0.05).

Compared with the normoxia control group, female rats in PQQ drug dose group II and PQQ drug dose group III have lower serum HDL cholesterol values (P<0.05), the serum HDL cholesterol values of the acetazolamide positive drug control group and the normoxazinamide group are increased (P<0.05), and the other groups have no statistically significant difference (P>0.05). Compared with the hypoxia model group, the serum HDL cholesterol value of female rats in the PQQ drug dose group III is decreased (P<0.05), and the serum HDL cholesterol value of the acetazolamide positive drug control group is increased (P<0.05), and the other groups have no statistically significant difference (P>0.05).

Compared with the normoxia control group, the serum low-density lipoprotein cholesterol values of female rats in the hypoxia model group and the drug intervention group after hypoxia exposure are decreased (P<0.05), and the serum low-density lipoprotein cholesterol values in the normoxia PQQ drug group and the acetazolamide group have no statistically significant difference (P>0.05). Compared with the hypoxia model group, the LDL cholesterol value of female rats in the acetazolamide positive drug control group is increased (P<0.05), and there is no statistically significant difference in the other groups (P>0.05).

### 7.5 Effects of PQQ on liver function of rats exposed to acute altitude hypoxia

**Table 24 Effects of PQQ on liver function of male rats exposed to acute altitude hypoxia**

| Group | Glutamic-pyruvic transaminase (U) | Glutamic oxalacetic transaminase (U) | Total bilirubin(µmol/L) |
|---|---|---|---|
| Normoxia control group (equal volume of water) | 37.11±6.01 | 82.13±15.34 | 0.46±0.24 |
| Hypoxia model group(equal volume of water) | 42.33±9.80 | 96.44±13.81 | 0.59±0.24 |
| PQQ drug dose group I(0.91mg/kg) | 36.63±8.30 | 79.17±15.66# | 0.51±0.12 |
| PQQ drug dose group II(1.83mg/kg) | 40.63±5.18 | 85.80±13.80 | 0.59±0.12 |
| PQQ drug dose group III(3.66mg/kg) | 42.50±4.93 | 99.00±15.52 | 0.60±0.15 |
| PQQ drug dose group IV(7.31mg/kg) | 43.13±8.92 | 94.40±12.28 | 0.46±0.15 |
| PQQ drug dose group V(14.63mg/kg) | 48.25±8.86* | 109.00±11.68** | 0.53±0.15 |
| Acetazolamide positive drug control group(0.11g/kg) | 44.00±3.56* | 77.57±6.80## | 0.15±0.16 |

| | | | |
|---|---|---|---|
| *p<0.05, **p<0.01 VS normoxia control group, #p<0.05, ##p<0.01 VS hypoxia model group | | | |

It can be seen from Table 24 that compared with the normoxia control group, the serum glutamic-pyruvic transaminase activity of male rats in the PQQ drug dose group V and the acetazolamide positive drug control group is increased (P<0.05), and the other groups have no statistically significant difference (P>0.05). Compared with the hypoxia model group, there is no statistically significant difference in the alanine aminotransferase activity of male rats in each treatment group (P>0.05).

Compared with the normoxia control group, the serum glutamic oxalacetic transaminase activity of male rats in PQQ drug dose group V is increased (P<0.05), and there is no statistically significant difference in the other groups (P>0.05). Compared with the hypoxia model group, the serum glutamic oxalacetic transaminase activity of male rats in the PQQ drug dose group I and the acetazolamide positive drug control group decreased, and there was a statistically significant difference (P<0.05), while the other groups had no statistically significant difference (P>0.05).

Compared with the normoxia control group and hypoxia model group, there is no statistically significant difference in total bilirubin of male rats in each group (P>0.05).

**Table 25 Effect of PQQ on liver function of famale rats exposed to acute altitude hypoxia**

| Group | Glutamic-pyruvic transaminase (U) | Glutamic oxalacetic transaminase (U) |
|---|---|---|
| Normoxia control group (equal volume of water) | 35.89±9.61 | 95.00±15.72 |
| Hypoxia model group(equal volume of water) | 33.33±5.17 | 78.00±17.02* |
| PQQ drug dose group I(0.91mg/kg) | 29.86±4.30 | 80.71±12.49 |
| PQQ drug dose group II(1.83mg/kg) | 27.86±6.28 | 84.71±18.97 |
| PQQ drug dose group III(3.66mg/kg) | 32.57±5.35 | 82.29±17.75 |
| PQQ drug dose group IV(7.31mg/kg) | 32.57±6.37 | 79.29±16.06 |
| PQQ drug dose group V(14.63mg/kg) | 36.57±8.52 | 83.00±20.18 |
| Acetazolamide positive drug control group(0.11g/kg) | 28.40±2.30 | 73.20±11.19* |
| Normoxia PQQ drug group(3.66mg/kg) | 37.14±4.45 | 120.29±11.90** |
| Normoxia acetazolamide group(0.11g/kg) | 41.67±10.97 | 102.33±11.02 |

| | | |
|---|---|---|
| *p<0.05, **p<0.01 VS normoxia control group, #p<0.05, ##p<0.01 VS hypoxia model group | | |

It can be seen from Table 25 that compared with the normoxia control group and the hypoxia model group, there is no statistically significant difference in the glutamic-pyruvic transaminase activity of female rats in each group (P>0.05).

Compared with the normoxia control group, the serum glutamic oxalacetic transaminase activity of female rats in the hypoxia model group and the acetazolamide positive drug control group decreased (P<0.05), and the normoxia PQQ drug group is increased (P<0.05). Compared with the hypoxia model group, there is no statistically significant difference in serum glutamic oxalacetic transaminase activity in each drug intervention group (P>0.05).

**7.6 Effects of PQQ on renal function in rats exposed to acute altitude hypoxia Table 26 Effects of PQQ on renal function in male rats exposed to acute altitude hypoxia**

| Group | Serum urea nitrogen (mmol/L) | Creatinine(µmol/L ) | Uric acid (µmol/L ) |
|---|---|---|---|
| Normoxia control group | 5.37±0.62 | 22.43±2.06 | 161.86±9.21 |
| (equal volume of water) | | | |
| Hypoxia model group(equal volume of water) | 6.10±1.08 | 22.60±5.51 | 180.33±17.30* |
| PQQ drug dose group I(0.91mg/kg) | 5.13±0.75 | 18.44±1.46** | 134.25±19.08**## |
| PQQ drug dose group II(1.83mg/kg) | 5.44±1.13 | 21.59±3.31 | 142.60±10.78**## |
| PQQ drug dose group III(3.66mg/kg) | 6.37±1.64 | 21.90±2.88 | 171.38±14.92 |
| PQQ drug dose group IV(7.3 1mg/kg) | 5.91±0.86 | 22.40±1.53 | 202.16±17.13**# |
| PQQ drug dose group V(14.63mg/kg) | 6.23±1.36 | 22.49±3.00 | 182.22±15.80** |
| Acetazolamide positive drug control group(0.11g/kg) | 7.99±1.03**## | 25.01±2.48* | 81.86±11.05**## |

| | | | |
|---|---|---|---|
| *p<0.05, **p<0.01 VS normoxia control group, #p<0.05, ##p<0.01 VS hypoxia model group | | | |

It can be seen from Table 26 that compared with the normoxia control group and the hypoxia model group, the serum urea nitrogen content of the male rats in the acetazolamide positive drug control group is increased (P<0.05), and has no statistics significant difference in the other groups (P>0.05).

Compared with the normoxia control group, the serum creatinine value of male rats in the PQQ drug dose group I is decreased (P<0.05), and the serum creatinine value of the acetazolamide positive drug control group is increased (P<0.05), but has no statistics significant difference in the other groups (P>0.05). Compared with the hypoxia model group, there is no statistically significant difference in each group (P>0.05).

Compared with the normoxia control group, the serum uric acid levels of male rats in hypoxia model group, PQQ drug dose group IV and PQQ drug dose group V are increased (P<0.05). The serum uric acid levels of the PQQ drug dose group I, the drug dose group II and the acetazolamide positive drug control group are decreased, and there is a statistically significant difference (P<0.05). There is no statistically significant difference in the PQQ drug dose group III (P>0.05). Compared with the hypoxia model group, the serum uric acid levels of male rats in the PQQ drug dose group I, drug dose group II and the acetazolamide positive drug control group are decreased, and there is a statistically significant difference (P<0.05). The serum uric acid level in the PQQ drugs dose group IV is increased (P<0.05), and there is no statistically significant difference in the other groups (P>0.05).

**Table 27 Effects of PQQ on renal function in female rats exposed to acute altitude hypoxia**

| Group | Serum urea nitrogen (mmol/L) | Creatinine(µmol/L) | Uric acid (µmol/L) |
|---|---|---|---|
| Normoxia control group (equal volume of water) | 5.76±0.74 | 17.81±0.90 | 165.89±19.35 |
| Hypoxia model group(equal volume of water) | 6.10±0.64 | 14.88±1.46** | 147.78±12.38* |
| PQQ drug dose group I(0.91mg/kg) | 6.85±0.90* | 14.67±1.46** | 163.43±12.51# |
| PQQ drug dose group II(1.83mg/kg) | 5.98±0.62 | 14.23±1.90** | 152.71±16.58 |
| PQQ drug dose group III(3.66mg/kg) | 5.79±0.62 | 12.71±1.98**# | 151.14±18.48 |
| PQQ drug dose group IV(7.31mg/kg) | 6.00±0.98 | 16.06±1.19** | 152.43±25.28 |
| PQQ drug dose group V(14.63mg/kg) | 5.86±0.54 | 13.51±1.33** | 155.33±21.56 |
| Acetazolamide positive drug control group(0.11g/kg) | 8.02±1.05**## | 18.78±1.96## | 77.80±17.31*## |
| Normoxia PQQ drug group(3.66mg/kg) | 6.23±0.36 | 19.37±4.32 | 193.57±20.80* |
| Normoxia acetazolamide group(0.11g/kg) | 7.86±0.49** | 19.67±0.32** | 82.00±15.10** |

| | | | |
|---|---|---|---|
| *p<0.05, **p<0.01 VS normoxia control group, #p<0.05, ##p<0.01 VS hypoxia model group | | | |

It can be seen from Table 27 that compared with the normoxia control group, the serum urea nitrogen levels of female rats in the PQQ drug dose group I, the acetazolamide positive drug control group and the normoxazolamide group after hypoxia exposure are increased (P <0.05), there is no statistically significant difference in other groups (P>0.05). Compared with the hypoxia model group, the serum urea nitrogen level of female rats in the acetazolamide positive drug control group is statistically significantly increased (P<0.05), and there is no statistically significant difference in the other groups (P >0.05).

Compared with the normoxia control group, the serum creatinine values of female rats in the hypoxia model group and the PQQ drug dose groups after hypoxia exposure are decreased, and there is a statistically significant difference (P<0.05). The creatinine value in the acetazolamide positive drug control group is increased (P<0.05), and there is no statistically significant difference in other groups (P>0.05). Compared with the hypoxia model group, the serum creatinine value of female rats in the PQQ drug dose group III is decreased, and there is a statistically significant difference (P<0.05), the serum creatinine value of the acetazolamide positive drug control group is increased (P< 0.05), there is no statistically significant difference in other groups (P>0.05).

Compared with the normoxia control group, the serum uric acid levels in the hypoxia model group, the acetazolamide positive drug control group and the normoxazolamide group are decreased serum decreased, and there is a statistically significant difference (P<0.05). The serum uric acid level in the PQQ drug group is increased (P<0.05), and there is no statistically significant difference in the other groups (P>0.05). Compared with the hypoxia model group, the serum uric acid level of male rats in the acetazolamide positive drug control group is decreased (P<0.05), and the serum uric acid level of PQQ drug dose group I is slightly increased (P<0.05), and the other groups have no statistically significant difference (P>0.05).

### 7.7 Effects of PQQ on myocardial enzyme activity in rats exposed to acute altitude hypoxia

**Table 28 Effects of PQQ on myocardial enzyme activity in male rats exposed to acute altitude hypoxia**

| Group | Lactate dehydrogenase (U/L) | Creatine Kinase (U/L) | α-hydroxybutyrate dehydrogenase(U/L) |
|---|---|---|---|
| Normoxia control group (equal volume of water) | 532.00±315.79 | 524.11±222.26 | 218.67±129.36 |
| Hypoxia model group(equal volume of water) | 449.22±261.12 | 396.67±172.01 | 184.00±105.80 |
| PQQ drug dose group I(0.91mg/kg) | 463.38±364.37 | 443.63±260.18 | 218.00±173.09 |
| PQQ drug dose group II(1.83mg/kg) | 650.13±573.72 | 544.63±418.71 | 286.75±260.98 |
| PQQ drug dose group III(3.66mg/kg) | 588.13±360.36 | 544.50±243.30 | 256.63±154.55 |
| PQQ drug dose group IV(7.3 1mg/kg) | 767.38±546.88 | 640.25±333.16 | 325.63±238.56 |
| PQQ drug dose group V(14.63mg/kg) | 510.44±275.48 | 510.89±240.61 | 218.67±118.08 |
| Acetazolamide positive drug control group(0.11g/kg) | 303.86±174.67 | 352.43±138.11 | 126.29±67.51 |

| | | | |
|---|---|---|---|
| *p<0.05, **p<0.01 VS normoxia control group, #p<0.05, ##p<0.01 VS hypoxia model group | | | |

It can be seen from Table 28 that compared with the normoxia control group and the hypoxia model group, there is no statistically significant difference in the lactate dehydrogenase of male rats in each group (P>0.05).

Compared with the normoxia control group and hypoxia model group, there is no statistically significant difference in the creatine kinase of male rats in each group (P>0.05).

Compared with the normoxia control group and hypoxia model group, there is no statistically significant difference in the α-hydroxybutyrate dehydrogenase of male rats in each group (P>0.05).

**Table 29 Effects of PQQ on myocardial enzyme activity in female rats exposed to acute altitude**

| Group | Lactate dehydrogenase (U/L) | Creatine Kinase (U/L) | α-hydroxybutyrate dehydrogenase(U/L) |
|---|---|---|---|
| Normoxia control group (equal volume of water) | 774.78±378.72 | 712.89±166.80 | 376.11±132.67 |
| Hypoxia model group(equal volume of water) | 494.11±333.54 | 437.11±291.80* | 209.11±151.30* |
| PQQ drug dose group I(0.91mg/kg) | 627.43±253.66 | 505.43±202.03* | 270.28±111.79 |
| PQQ drug dose group II(1.83mg/kg) | 650.00±338.13 | 508.57±235.83 | 286.71±154.38 |
| PQQ drug dose group III(3.66mg/kg) | 544.14±314.26 | 459.14±239.34* | 233.43±147.72 |
| PQQ drug dose group IV(7.3 1mg/kg) | 499.71±320.46 | 454.86±241.51* | 210.14±140.24* |
| PQQ drug dose group V(14.63mg/kg) | 496.86±259.65 | 439.86±222.18* | 208.86±112.12* |
| Acetazolamide positive drug control group(0.11g/kg) | 534.00±179.23 | 503.40±137.62* | 230.20±75.49* |
| Normoxia PQQ drug group(3.66mg/kg) | 1199.14±204.93** | 1024.00±313.62* | 537.29±92.21* |
| Normoxia | 998.67±285.56 | 697.67±222.15 | 430.33±134.10 |
| acetazolamide group(0.11g/kg) | | | |

| | | | |
|---|---|---|---|
| *p<0.05, **p<0.01 VS normoxia control group, #p<0.05, ##p<0.01 VS hypoxia model group | | | |

It can be seen from Table 29 that compared with the normoxia control group, the lactate dehydrogenase activity of the normoxia PQQ drug group is increased (P<0.05), and there is no statistically significant difference in the other groups (P>0.05). Compared with the hypoxia model group, there is no statistically significant difference in each group (P>0.05).

Compared with the normoxia control group, the serum creatine kinase activity of female rats in the hypoxia model group and the drug group after each hypoxia exposure is decreased (P<0.05), and the serum creatine kinase activity of the normoxia PQQ drug group is increased (P <0.05), there is no statistically significant difference in other groups (P>0.05). Compared with the hypoxia model group, there is no statistically significant difference in each group (P>0.05).

Compared with the normoxia control group, the serum α-hydroxybutyrate dehydrogenase activity of female rats in the hypoxia model group, the drug dose group IV, the drug dose group V and the positive acetazolamide control group after hypoxia exposure is decreased, there is statistically significant difference (P<0.05). The serum α-hydroxybutyrate dehydrogenase activity in the normoxia PQQ drug group is increased (P<0.05), and there is no statistically significant difference in other groups (P>0.05). Compared with the hypoxia model group, there is no statistically significant difference in each group (P>0.05).

### 7.8 Effects of PQQ on serum homocysteine in rats exposed to acute altitude hypoxia

**Table 30 Effects of PQQ on serum homocysteine in male rats exposed to acute altitude hypoxia**

| Group | Serum homocysteine (µmol/L) |
|---|---|
| Normoxia control group (equal volume of water) | 9.09±2.29 |
| Hypoxia model group(equal volume of water) | 11.98±2.28* |
| PQQ drug dose group I(0.91mg/kg) | 11.18±2.09 |
| PQQ drug dose group II(1.83mg/kg) | 13.24±3.87* |
| PQQ drug dose group III(3.66mg/kg) | 13.65±3.24** |
| PQQ drug dose group IV(7.31mg/kg) | 11.48±2.36 |
| PQQ drug dose group V(14.63mg/kg) | 12.78±2.87** |
| Acetazolamide positive drug control group(0.11g/kg) | 11.13±1.67 |

| | |
|---|---|
| *p<0.05, **p<0.01 VS normoxia control group, #p<0.05, ##p<0.01 VS hypoxia model group | |

It can be seen from Table 30 that compared with the normoxia control group, the serum homocysteine levels of male rats in the hypoxia model group, PQQ drug dose group II, drug dose group III and drug dose group V are increased (P< 0.05), the other groups have no statistically significant differences (P>0.05). Compared with the hypoxia model group, there is no statistically significant difference in each drug intervention group (P>0.05).

**Table 31 Effects of PQQ on serum homocysteine in female rats exposed to acute altitude hypoxia**

| Group | Serum homocysteine (µmol/L ) |
|---|---|
| Normoxia control group (equal volume of water) | 9.82±1.29 |
| Hypoxia model group(equal volume of water) | 8.14±1.6* |
| PQQ drug dose group I(0.91mg/kg) | 9.43±1.4 |
| PQQ drug dose group II(1.83mg/kg) | 10.66±2.13# |
| PQQ drug dose group III(3.66mg/kg) | 9.63±1.97 |
| PQQ drug dose group IV(7.31mg/kg) | 8.6±1.72 |
| PQQ drug dose group V(14.63mg/kg) | 8.11±1.37* |
| Acetazolamide positive drug control group(0.11g/kg) | 9.54±0.98 |
| Normoxia PQQ drug group(3.66mg/kg) | 9.76±1.16 |
| Normoxia acetazolamide group(0.11g/kg) | 9.33±1.16 |

| | |
|---|---|
| *p<0.05, **p<0.01 VS normoxia control group, #p<0.05, ##p<0.01 VS hypoxia model group | |

It can be seen from Table 31 that compared with the normoxia control group, the serum homocysteine levels of female rats in the hypoxia model group and the PQQ drug dose group V after hypoxia exposure are decreased (P<0.05), there is no statistically significant difference in each drug intervention group(P>0.05). Compared with the hypoxia model group, the serum homocysteine level of female rats in the PQQ drug dose group II after hypoxia exposure is increased (P<0.05), and there is no statistically significant difference in the other groups (P> 0.05).

### 7.9 Effects of PQQ on endothelial function in rats exposed to acute altitude hypoxia

**Table 32 Effects of PQQ on endothelial function in male rats exposed to acute altitude hypoxia**

| Group | Endothelin-1 (ng/mL) | Nitric oxide (µmol/L) |
|---|---|---|
| Normoxia control group (equal volume of water) | 45.25±3.11 | 5.39±2.3 |
| Hypoxia model group(equal volume of water) | 46.14±3.20 | 5.33±1.42 |
| PQQ drug dose group I(0.91mg/kg) | 45.35±4.24 | 5.28±0.71 |
| PQQ drug dose group II(1.83mg/kg) | 42.59±1.45# | 5.15±2.11 |
| PQQ drug dose group III(3.66mg/kg) | 48.14±3.13 | 5.35±1.35 |
| PQQ drug dose group IV(7.3 1mg/kg) | 43.60±0.94 | 6.20±2.49 |
| PQQ drug dose group V(14.63mg/kg) | 42.45±3.84 | 6.46±1.44 |
| Acetazolamide positive drug control group(0.11g/kg) | 42.41±0.7# | 3.52±1.25# |

| | | |
|---|---|---|
| *p<0.05, **p<0.01 VS normoxia control group, #p<0.05, ##p<0.01 VS hypoxia model group | | |

It can be seen from Table 32 that compared with the normoxia control group, there is no statistically significant difference in the serum endothelin-1 content of the hypoxia model group and each drug intervention group(P>0.05). Compared with the hypoxia model group, the serum endothelin-1 levels in the PQQ drug dose group II and the acetazolamide positive drug control group are decreased (P<0.05), and there is no statistically significant difference in the other groups (P>0.05).

Compared with the normoxia control group, there is no statistically significant difference in serum nitric oxide content of the hypoxia model group and the drug intervention groups (P>0.05). Compared with the hypoxia model group, the serum nitric oxide content of the acetazolamide positive drug control group is decreased (P<0.05), and there is no statistically significant difference in the other groups (P>0.05).

**Table 33 Effects of PQQ on endothelial function in female rats exposed to acute altitude hypoxia**

| Group | Endothelin-1 (ng/mL) | Nitric oxide (µmol /L) |
|---|---|---|
| Normoxia control group (equal volume of water) | 55.50±4.69 | 5.74±1.49 |
| Hypoxia model group(equal volume of water) | 49.59±5.92* | 6.03±2.28 |
| PQQ drug dose group 1(0.91mg/kg) | 64.75±2.72**## | 3.50±1.24**# |
| PQQ drug dose group II(1.83mg/kg) | 63.21±4.33**## | 4.47±1.35 |
| PQQ drug dose group III(3.66mg/kg) | 68.58±5.38**## | 6.19±2.06 |
| PQQ drug dose group IV(7.31mg/kg) | 65.44±14.06*# | 5.92±2.31 |
| PQQ drug dose group V(14.63mg/kg) | 65.26±6.60**## | 4.62±3.25 |
| Acetazolamide positive drug control group(0.11g/kg) | 55.66±6.39 | 4.96±3.13 |
| Normoxia PQQ drug group(3.66mg/kg) | 47.50±7.31* | 5.01±1.57 |
| Normoxia acetazolamide group(0.11g/kg) | 36.18±2.31** | 4.08±0.88 |

| | | |
|---|---|---|
| *p<0.05, **p<0.01 VS normoxia control group, #p<0.05, ##p<0.01 VS hypoxia model group | | |

It can be seen from Table 33 that compared with the normoxia control group, the serum endothelin-1 levels of female rats in the hypoxia model group, the normoxia PQQ drug group and the normoxazinamide group are decreased (P<0.05), the levels of serum endothelin-1 in each PQQ drug dose group after hypoxia exposure are increased, and has statistically significant (P<0.05), and there is no statistically significant difference in the acetazolamide positive drug control group (P>0.05). Compared with the hypoxia model group, the serum endothelin-1 levels in the PQQ medication group after hypoxia exposure are increased (P<0.05), and there is no statistically significant difference in the acetazolamide positive control group (P>0.05).

Compared with the normoxia control group and hypoxia model group, the serum nitric oxide content of female rats in the PQQ drug dose group I after hypoxia exposure is decreased (P<0.05), and there is no statistically significant difference in the other groups (P >0.05).

### 7.10 Effects of PQQ on oxidative stress indexes of rats with acute altitude hypoxia injury

**Table 34 Effect of PQQ on oxidative stress indexes of male rats with acute altitude hypoxia injury**

| Group | Superoxide dismutase (U/ml) | Total antioxidants (U/ml) | Malonaldehyde (nmol/L) |
|---|---|---|---|
| Normoxia control group (equal volume of water) | 176.02±2.97 | 2.48±0.79 | 2.09±1.27 |
| Hypoxia model group(equal volume of water) | 173.43±4.44 | 2.05±0.68 | 3.29±1.59 |
| PQQ drug dose group I(0.91mg/kg) | 196.26±4.15**## | 3.17±1.40 | 2.79±0.62 |
| PQQ drug dose group II(1.83mg/kg) | 193.38±4.53**## | 2.77±1.10 | 2.89±1.09 |
| PQQ drug dose group III(3.66mg/kg) | 189.59±5.46**## | 3.81±0.97*# | 3.9±1.39 |
| PQQ drug dose group IV(7.3 1mg/kg) | 186.20±6.32**## | 3.62±1.94 | 3.15±0.99 |
| PQQ drug dose group V(14.63mg/kg) | 179.58±6.32 | 1.49±2.30 | 3.59±1.15* |
| Acetazolamide positive drug control group(0.11g/kg) | 182.51±4.46*## | 1.23±1.93 | 3.24±1.52 |

| | | | |
|---|---|---|---|
| *p<0.05, **p<0.01 VS normoxia control group, #p<0.05, ##p<0.01 VS hypoxia model group | | | |

It can be seen from Table 34 that compared with the normoxia control group and the hypoxia model group, the serum superoxide dismutase activity of male rats in the PQQ drug dose group I, II, III, and IV and the acetazolamide positive drug control group is increased (P<0.05), there is no statistically significant difference in drug dose group V (P>0.05).

Compared with the normoxia control group and the hypoxia model group, the serum total antioxidant value of male rats in the PQQ drug dose group III is increased (P<0.05), and there is no statistically significant difference in the other groups (P>0.05).

Compared with the normoxia control group, the serum malondialdehyde value of male rats in the PQQ drug dose group V is increased (P<0.05), and there is no statistically significant difference in the other groups (P>0.05). Compared with the hypoxia model group, there is no statistically significant difference in each group (P>0.05).

**Table 35 Effects of PQQ on oxidative stress indexes of female rats with acute altitude hypoxia injury**

| Group | Superoxide dismutase (U/ml) | Total antioxidants (U/ml) | Endo Dialdehyde(nmol/L) |
|---|---|---|---|
| Normoxia control group (equal volume of water) | 53.32±2.39 | 11.19±0.71 | 13.09±0.85 |
| Hypoxia model group(equal volume | 52.84±2.55 | 10.66±0.93 | 13.01±0.79 |
| of water) | | | |
| PQQ drug dose group I(0.91mg/kg) | 66.11±4.18**## | 10.52±1.08 | 15.07±1.91*# |
| PQQ drug dose group II(1.83mg/kg) | 60.79±2.56**## | 10.06±0.77** | 13.96±1.33 |
| PQQ drug dose group III(3.66mg/kg) | 56.82±3.80*# | 10.84±0.47 | 13.73±0.27 |
| PQQ drug dose group IV(7.3 1mg/kg) | 56.65±2.97*# | 10.37±0.75* | 14.14±2.68 |
| PQQ drug dose group V(14.63mg/kg) | 56.16±3.64# | 10.59±1.65 | 14.81±1.37**# |
| Acetazolamide positive drug control group(0.11g/kg) | 57.11±3.40*# | 10.36±0.81 | 13.09±2.13 |
| Normoxia PQQ drug group(3.66mg/kg) | 52.21±1.23 | 11.28±0.83 | 14.39±0.85** |
| Normoxia acetazolamide group(0.11g/kg) | 50.88±3.34 | 10.31±0.20 | 14.12±0.32 |

| | | | |
|---|---|---|---|
| *p<0.05, **p<0.01 VS normoxia control group, #p<0.05, ##p<0.01 VS hypoxia model group | | | |

It can be seen from Table 35 that compared with the normoxia control group, the serum superoxide dismutase activity of female rats in the PQQ drug dose group I, II, III, and IV and the acetazolamide positive drug control group after hypoxia exposure is increased (P<0.05), there is no statistically significant difference in the drug dose group V, the normoxia PQQ drug group and the normoxia acetazolamide group (P>0.05). Compared with the hypoxia model group, the superoxide dismutase activity of female rats in each PQQ drug dose group after hypoxia exposure is increased, and there is a statistically significant difference (P<0.05).

Compared with the normoxia control group, the total antioxidants value of female rats in PQQ drug dose group II and drug dose group IV after hypoxia exposure is increased statistically (P<0.05), but has no statistics significant difference in other groups (P>0.05). Compared with the hypoxia model group, there is no statistically significant difference in each group (P>0.05).

Compared with the normoxia control group, the serum malondialdehyde level of female rats in the PQQ drug dose group I, drug dose group V and the normoxia PQQ drug group after hypoxia exposure is increased significantly (P<0.05) , the other groups had no statistically significant difference (P>0.05). Compared with the hypoxia model group, the serum malondialdehyde value of female rats in PQQ drug dose group I and drug dose group V is increased (P<0.05), and there is no statistically significant difference in other groups (P>0.05) .

### 7.11 Effects of PQQ on serum energy metabolism indexes in rats with acute altitude hypoxia injury

**Table 36 Effects of PQQ on serum energy metabolism indexes in male rats with acute altitude hypoxia injury**

| Group | ATP (g/ml) | Lactic acid (ng/L) |
|---|---|---|
| Normoxia control group (equal volume of water) | 168.9±16.37 | 171.99±11.17 |
| Hypoxia model group(equal volume of water) | 180.5±17.85 | 182.06±17.02 |
| PQQ drug dose group I(0.91mg/kg) | 185.81±28.52 | 188.70±29.68 |
| PQQ drug dose group II(1.83mg/kg) | 184.99±17.98 | 177.33±19.55 |
| PQQ drug dose group III(3.66mg/kg) | 188.84±24.99 | 189.12±24.64 |
| PQQ drug dose group IV(7.3 1mg/kg) | 178.23±9.97 | 172.64±12.25 |
| PQQ drug dose group V(14.63mg/kg) | 188.83±22.67 | 180.81±24.64 |
| Acetazolamide positive drug control group(0.11g/kg) | 161.13±15.39 | 161.46±6.94# |

| | | |
|---|---|---|
| *p<0.05, **p<0.01 VS normoxia control group, #p<0.05, ##p<0.01 VS hypoxia model group | | |

It can be seen from Table 36 that compared with the normoxia control group and hypoxia model group, the serum ATP content of male rats in each group has no statistically significant difference (P>0.05).

Compared with the normoxia control group, the serum lactic acid content of male rats in each group has no statistically significant difference (P>0.05). Compared with the hypoxia model group, the serum lactic acid content of male rats in the acetazolamide positive drug control group is decreased (P<0.05), and there is no statistically significant difference in the other groups (P>0.05).

**Table 37 Effect of PQQ on serum energy metabolism indexes in female rats with acute altitude hypoxia injury**

| Group | ATP (g/ml) | Lactic acid (ng/L) |
|---|---|---|
| Normoxia control group (equal volume of water) | 166.83±19.73 | 174.08±20.52 |
| Hypoxia model group(equal volume of water) | 166.05±23.55 | 116.1±20.02** |
| PQQ drug dose group I(0.91mg/kg) | 202.36±8.59**## | 222.06±12.56**## |
| PQQ drug dose group II(1.83mg/kg) | 200.55±20.92**## | 221.04±14.36**## |
| PQQ drug dose group III(3.66mg/kg) | 212.82±8.82**## | 242.8±8.13**## |
| PQQ drug dose group IV(7.31mg/kg) | 210.73±10.57**## | 196.41±11.86*## |
| PQQ drug dose group V(14.63mg/kg) | 197.73±17.99**# | 173.09±24.20## |
| Acetazolamide positive drug control group(0.11g/kg) | 183.19±24.74 | 132.16±10.42** |
| Normoxia PQQ drug group(3.66mg/kg) | 149.69±24.29 | 116.73±13.32** |
| Normoxia acetazolamide group(0.11g/kg) | 138.28±13.97* | 100.54±7.13** |

| | | |
|---|---|---|
| *p<0.05, **p<0.01 VS normoxia control group, #p<0.05, ##p<0.01 VS hypoxia model group | | |

It can be seen from Table 37 that compared with the normoxia control group, the serum ATP content of the each PQQ drug dose group after hypoxia exposure is increased, and there is a statistically significant difference (P<0.05). Compared with the hypoxia model group, the serum ATP content of the each PQQ drug dose group after hypoxia exposure is increased(P<0.05), and there is no statistically significant difference in the acetazolamide positive drug control group (P>0.05). The serum ATP content of female rats in the normoxazolamide group is decreased, and there is a statistically significant difference (P<0.05).

Compared with the normoxia control group, the hypoxia model group, the Acetazolamide positive drug control group, the normoxia PQQ drug group and the normoxazolamide group have lower serum lactic acid levels, and has statistically significant (P <0.05). After hypoxia exposure, the serum lactic acid content of PQQ drug dose group I, II, III, IV is increased (P<0.05). The serum lactic acid content in drug dose group V compared with normoxia control group is not statistically significant difference(P>0.05). Compared with the hypoxia model group, the serum lactic acid content of each PQQ drug dose group after hypoxia exposure is increased, and there is a statistically significant difference (P<0.05), the serum lactic acid content in acetazolamide positive drug control group has no statistically significant difference (P>0.05).

### 7.12 Effects of PQQ on liver energy metabolism indexes of rats exposed to acute altitude hypoxia

**Table 38 Effects of PQQ on liver ATP and glycogen in male rats exposed to acute altitude hypoxia**

| Group | ATP (g/ml) | Glycogen (ng/ml) |
|---|---|---|
| Normoxia control group (equal volume of water) | 3.81±0.90 | 2.31±0.57 |
| Hypoxia model group(equal volume of water) | 3.38±0.56 | 2.03±0.35 |
| PQQ drug dose group I(0.91mg/kg) | 3.65±0.62 | 2.41±0.41 |
| PQQ drug dose group II(1.83mg/kg) | 3.71±0.30 | 2.23±0.17 |
| PQQ drug dose group III(3.66mg/kg) | 4.46±1.50 | 2.70±0.84# |
| PQQ drug dose group IV(7.3 1mg/kg) | 5.04±0.79**## | 2.86±0.43*## |
| PQQ drug dose group V(14.63mg/kg) | 4.09±0.49# | 2.55±0.28## |
| Acetazolamide positive drug control group(0.11g/kg) | 3.48±0.44 | 2.14±0.21 |

| | | |
|---|---|---|
| *p<0.05, **p<0.01 VS normoxia control group, #p<0.05, ##p<0.01 VS hypoxia model group | | |

It can be seen from Table 38 that compared with the normoxia control group, the ATP value in the liver of male rats in the PQQ drug dose group has a rising trend, and the increase in the content of the PQQ drug dose group IV has a statistically significant difference (P<0.05). Compared with the hypoxia model group, the ATP content in the liver of male rats in PQQ drug dose group IV and PQQ drug dose group V is increased (P<0.05), and there is no statistically significant difference in other groups (P>0.05).

Compared with the normoxia control group, the glycogen value in the liver of male rats in the PQQ drug dose group IV is increased (P<0.05), and there is no statistically significant difference in the other groups (P>0.05). Compared with the hypoxia model group, the glycogen value in the liver of male rats in PQQ drug dose group III, PQQ drug dose group IV, and PQQ drug dose group V is increased, has a statistically significant difference (P<0.05). There is no statistically significant difference in other groups (P>0.05).

**Table 39 Effects of PQQ on liver ATP and glycogen in female rats exposed to acute altitude hypoxia**

| Group | ATP (g/ml) | Glycogen (ng/ml) |
|---|---|---|
| Normoxia control group (equal volume of water) | 1.37±0.27 | 2.68±0.42 |
| Hypoxia model group(equal volume of water) | 1.87±0.36** | 3.5±0.57** |
| PQQ drug dose group I(0.91mg/kg) | 1.48±0.19# | 2.52±0.36## |
| PQQ drug dose group II(1.83mg/kg) | 1.58±0.27 | 2.89±0.57 |
| PQQ drug dose group III(3.66mg/kg) | 1.64±0.37 | 2.84±0.67 |
| PQQ drug dose group IV(7.3 1mg/kg) | 1.8±0.20** | 3.07±0.19* |
| PQQ drug dose group V(14.63mg/kg) | 1.87±0.26** | 3.62±0.38** |
| Acetazolamide positive drug control group(0.11g/kg) | 1.87±0.26** | 3.71±0.27** |
| Normoxia PQQ drug group(3.66mg/kg) | 1.62±0.13 | 2.89±0.28 |
| Normoxia acetazolamide group(0.11g/kg) | 1.39±0.54 | 2.75±1.13 |

| | | |
|---|---|---|
| *p<0.05, **p<0.01 VS normoxia control group, #p<0.05, ##p<0.01 VS hypoxia model group | | |

It can be seen from Table 39 that compared with the normoxia control group, the ATP content in the liver of female rats in the hypoxia model group, post-hypoxic exposure PQQ drug dose group IV, PQQ drug dose group V and the acetazolamide positive drug control group is increased (P<0.05), and there is no statistically significant difference in other groups (P>0.05). Compared with the hypoxia model group, the ATP content in the liver of female rats in the PQQ drug dose group I is decreased (P<0.05), and there is no statistically significant difference in the other groups (P>0.05).

Compared with the normoxia control group, the glycogen value in the liver of female rats in the hypoxia model group, PQQ drug dose group IV, PQQ drug dose group V and the acetazolamide positive drug control group is increased, and has statistically significant difference (P<0.05), there is no statistically significant difference in other groups (P>0.05). Compared with the hypoxia model group, the glycogen value in the liver of female rats in PQQ drug dose group I after hypoxia exposure is decreased (P<0.05), and there is no statistically significant difference in the other groups (P>0.05).

### 7. 13 Exhaustive swimming experiment on effects of PQQ on the working ability of mice under altitude hypoxia conditions

Exhaustive swimming experiment on the effect of PQQ on the working ability of mice under the condition of high altitude hypoxia

**Table 40 Changes of body weight of male mice in exhaustive swimming experiment**

| Group | male (g) | |
|---|---|---|
| | 1 day | 7 days |
| Negative control (equal volume of water) | 21.08±1.01 | 28.11±2.18 |
| Acetazolamide positive drug control group(0.16g/kg) | 21.28±1.38 | 23.05±4.41** |
| PQQ drug dose group I(1.32mg/kg) | 22.02±1.51 | 24.17±3.03** |
| PQQ drug dose group II(2.64mg/kg) | 21.19±1.25 | 25.56±2.02* |
| PQQ drug dose group III(5.28mg/kg) | 20.89±1.11 | 25.92±2.54* |
| PQQ drug dose group IV(10.56mg/kg) | 21.55±0.93 | 25.50±3.80 |
| PQQ drug dose group V(21.12mg/kg) | 20.85±1.15 | 26.36±4.29 |

| | | |
|---|---|---|
| *p<0.05, **p<0.01 VS negative control group | | |

It can be seen from Table 40 that compared with the negative control group, there is no statistically significant difference in the body weight of male mice before gavage administration (P>0.05). After intragastric administration for7 days, compared with the negative control group, the weight of male mice in the acetazolamide positive drug control group, the PQQ drug dose group I, the PQQ drug dose group II and the PQQ drug dose group III is decreased, there is statistically significant difference (P<0.05); and there is no statistically significant difference of the PQQ drug dose groups (P>0.05).

### 8. Summary of experimental results

### 8.1 Experiment of PQQ anti-acute altitude hypoxia injury in rats

### 8.1.1 Experiment of PQQ anti-acute altitude hypoxia injury in male rats

PQQ intervention for 7 days did not affect the weight of male rats, and the administration of acetazolamide could cause the weight of male rats decreased.

Acute hypoxia exposure has no significant effects on the blood glucose level of male rats, and the intervention of PQQ and acetazolamide has no significant effects on the blood glucose level of male rats.

Acute hypoxic exposure has no significant effects on the serum total protein content of male rats. Low-dose PQQ intervention can slightly reduce the serum total protein content of male rats exposed to hypoxia. High-dose PQQ interferes with the serum total protein content in rats is the same as that of the hypoxia model group. The effect of acetazolamide on the serum total protein content of male rats exposed to hypoxia is not found. Hypoxia exposure can cause a slight increase in serum albumin content, and low-dose PQQ intervention can reduce the effect of hypoxia exposure on the increase in serum albumin content in male rats. The effect of acetazolamide on the serum total protein content of male rats exposed to hypoxia is not found.

Acute hypoxia exposure has no significant effects on triglycerides in male rats, and the effect of PQQ and acetazolamide intervention on triglycerides in rats exposed to hypoxia is not found. Acute hypoxia exposure does not affect the content of total cholesterol and high-density lipoprotein cholesterol, but can slightly reduce the content of low-density lipoprotein cholesterol. PQQ low-dose intervention has the effects of reducing the amount of total cholesterol and high-density lipoprotein cholesterol, and has no significant effects on the amount of low density lipoprotein cholesterol, the intervention of acetazolamide has no significant effect on the above indicators.

Acute hypoxic exposure has no significant effects on glutamic-pyruvic transaminase, and the affect of PQQ intervention on serum glutamic-pyruvic transaminase, glutamic oxalacetic transaminase and total bilirubin in male rats exposed to acute hypoxia is not found. Acetazolamide has the effects of increasing serum glutamic oxalacetic transaminase in hypoxia exposed male rats, and has no significant effects on glutamic pyruvic transaminase and total bilirubin.

Acute hypoxia exposure has no significant effect on serum urea nitrogen and creatinine in male rats, but can cause a slight increase in uric acid content. PQQ intervention has no significant effect on serum urea nitrogen and creatinine in hypoxia exposed male rats, and low-dose intervention has the effects of reduce serum uric acid content. Acetazolamide can reduce the serum uric acid level of hypoxia exposed rats and increase the serum urea nitrogen content, and has no significant effect on serum creatinine;

Acute hypoxia exposure has no significant effects on the activities of serum lactate dehydrogenase, creatine kinase and α-hydroxybutyrate dehydrogenase in male rats. The effects of PQQ and acetazolamide intervention on serum lactate dehydrogenation creatine kinase, and α-hydroxybutyrate dehydrogenase in hypoxia exposed male rats has not been found.

Acute hypoxia exposure can slightly increase the serum homocysteine level of male rats. The effects of PQQ and Acetazolamide intervention on serum homocysteine of hypoxia exposed male rats have been not found.

Acute hypoxia exposure has no significant effect on serum endothelin-1 and nitric oxide levels in male rats. PQQ intervention has no significant effects on the above indicators. Acetazolamide has the effects of slight reduction serum endothelin-1 and nitric oxide content in hypoxia exposed male rats.

Acute hypoxia exposure has no significant effects on serum superoxide dismutase, total antioxidants, and malondialdehyde in male rats. PQQ intervention can increase the activity of serum superoxide dismutase in hypoxia exposed male rats, and have no significant effect to serum total antioxidants and malondialdehyde. Acetazolamide can also have the effects of increasing the activity of serum superoxide dismutase in male rats, and has no significant effects on serum total antioxidants and malondialdehyde.

Acute hypoxic exposure has no significant effects on serum ATP and lactic acid content in male rats. PQQ intervention has no significant effects on the above indicators in male rat serum. Acetazolamide intervention has no significant effects on serum ATP and has the effects of reducing serum lactic acid content.

Acute hypoxia exposure has no significant effects on liver ATP and glycogen content of male rats. PQQ intervention can increase liver ATP and glycogen content of male rats. Acetazolamide intervention has no significant effects on liver ATP and glycogen content of male rats.

### 8.1.2 Experiment of PQQ against acute altitude hypoxia injury in female rats

PQQ intervention for 7 days did not affect the body weight of female rats, and Acetazolamide had no significant effects on the body weight of female rats.

Acute hypoxia exposure can reduce the serum blood glucose level of female rats. PQQ intervention has the effects of increasing blood glucose in acute hypoxia exposed female rats. Acetazolamide intervention has no significant effects on the increase of blood glucose under acute hypoxia exposure. The normoxia PQQ intervention had no significant effects on the blood glucose of female rats. The intervention of the normoxia acetazolamide group has the effects of lowering the blood glucose of female rats.

Acute hypoxic exposure has no significant effects on the serum total protein content of female rats. Low-dose PQQ intervention can slightly reduce the serum total protein content of hypoxia exposed female rats. High-dose PQQ interferes serum total protein content in rats is the same as the hypoxia model group. The effects of acetazolamide on serum total protein content of female rats has been not found. Hypoxic exposure can cause a slight increase in serum albumin, and low-dose PQQ intervention can reduce the effect of serum albumin in female rats increase caused by hypoxic exposure. Acetazolamide intervention also has this effects. The normoxia PQQ intervention has no significant effects on the total serum protein content of female rats. normoxia acetazolamide can slightly increase the serum albumin content of female rats.

Acute hypoxia exposure can reduce the content of total cholesterol and low-density lipoprotein cholesterol in female rats, without affecting the content of high-density lipoprotein cholesterol. PQQ intervention has no significant effects on the above indicators. Acetazolamide intervention has the effects of increasing the content of total cholesterol, low-density lipoprotein cholesterol and high-density lipoprotein cholesterol. Noroxic PQQ intervention has no significant effects on the above indicators. Normoxia acetazolamide has no significant effects on the amount of low-density lipoprotein cholesterol, and can increase the total cholesterol and high-density lipoprotein cholesterol content of female rats.

Acute hypoxia exposure has no significant effects on glutamic-pyruvic transaminase in female rats, and can slightly reduce the activity of glutamic oxalacetic transaminase. The affect of PQQ intervention on the serum glutamic-pyruvic transaminase and glutamic oxalacetic transaminase activities of acute hypoxia exposed female rats has been not found. Noroxic PQQ intervention has the effects of slightly increasing the activity of aspartate aminotransferase, but has no significant effect on glutamic-pyruvic transaminase. Normoxia acetazolamide has no significant effects on the above indicators.

Acute hypoxia exposure has no significant effects on serum urea nitrogen in female rats, and can cause a slight decreased in content of creatinine and uric acid. PQQ intervention has no significant effects on serum urea nitrogen, creatinine and uric acid in hypoxia exposed female rats. Acetazolamide has the effects of decreasing serum uric acid level in hypoxia exposed female rats, and increasing the serum urea nitrogen and creatinine value. The normoxia PQQ intervention has no significant effects on the above indicators. Normoxia acetazolamide has the effects of reducing the serum uric acid level of female rats and increasing the serum urea nitrogen and creatinine values.

Acute hypoxia exposure can reduce the activity of serum creatine kinase and α-hydroxybutyrate dehydrogenase in female rats, and has no significant effects on the activity of serum lactate dehydrogenase. The significant effects of PQQ intervention on the activity of serum lactic dehydrogenase, creatine kinase, and α-hydroxybutyrate dehydrogenase has been not found. Acetazolamide has no significant effects on the above indicators. The normoxia PQQ intervention has the effects of increasing the above indicators. Normoxia acetazolamide has no significant effects on the above indicators.

Acute hypoxia exposure can slightly reduce the level of homocysteine in female rats. PQQ and acetazolamide intervention have no significant effects on serum homocysteine in hypoxia exposed female rats. The intervention of normoxia PQQ and acetazolamide had no significant effects on the above indicators.

Acute hypoxic exposure can reduce the serum endothelin-1 content of female rats, but has not been found to have a significant impact on the nitric oxide content. PQQ intervention can increase the serum endothelin-1 content of rats after acute hypoxia exposure, and has no significant effect to nitric oxide. Acetazolamide intervention has no significant effects on the above indicators. The intervention of normoxia PQQ and acetazolamide can reduce the serum endothelin-1 content of female rats, and no significant effects on the content of nitric oxide has been found.

Acute hypoxia exposure has no significant effects on serum superoxide dismutase, total antioxidants, and malondialdehyde in female rats. PQQ intervention can increase serum superoxide dismutase activity in hypoxia exposed female rats, and have no significant effects on serum total antioxidants and malondialdehyde. Acetazolamide can also have the effects of increasing the activity of serum superoxide dismutase, and has no significant effects on serum total antioxidants and malondialdehyde. Noroxic PQQ intervention can slightly increase the effects of malondialdehyde in female rats, and has no significant effects on serum superoxide dismutase and total antioxidants. Noroxazepine has no significant effects on the above indicators.

Acute hypoxia exposure has no significant effects on serum ATP in female rats, and has the effects of reducing serum lactic acid content. PQQ intervention has the effects of increasing serum ATP and lactic acid content in female rats. Acetazolamide has no significant effects on the above indicators. The normoxia PQQ intervention has the effects of reducing the serum lactic acid content, but has no significant effect on the serum ATP content. The Acetazolamide intervention has the effects of reducing the serum ATP and lactic acid content of female rats.

Acute hypoxia exposure can increase the liver ATP and glycogen content of female rats. After hypoxia exposure and the intervention of normoxia PQQ and Acetazolamide have no significant effects on the liver ATP and glycogen content of female rats.

### 8.2 Exhaustive swimming experiment of PQQ to improve the working ability of mice under altitude hypoxia conditions

Exhausted swimming test of male mice exposed to 6000 meters above sea level (administered 1.32mg/kg, 2.64mg/kg, 5.28mg/kg, 10.56mg/kg, 21.12mg/kg PQQ, administration for 7 days),. The results shows that the mice in the negative control group and the acetazolamide positive drug control group died earlier, the start time of exhaustion swimming death of mice in each dose group of PQQ is delayed compared with the above two groups. After exposure to 6000 meters above sea level for180 minutes, the mice in the negative control group and the acetazolamide positive drug control group are almost completely dead, and the mice in the each PQQ dose groups are completely dead about 400 minutes. Exhausted swimming survival time (minutes)of male mice in the negative control group, acetazolamide positive drug control group, and mice in the PQQ above five dose groups are 133.38±110.94, 130.64±79.46, 199.21±98.54, 273.10±63.07, 173.15±116.32, 195.04±59.81, 263.20±48.27. The increasing rate of survival time was 0%, 2.10%, 52.49%, 109.05%, 32.54%, 49.30%, 101.47%. The results of this experiment suggest that the administration of PQQ can prolong the survival time of mice after exhausting swimming, and has the effects of improving the working ability of mice under high altitude hypoxia exposure conditions.

The present invention is illustrated by the above examples, but it should be understood that the present invention is not limited to the specific examples and implementations described herein. The purpose of including these specific examples and embodiments here is to help those skilled in the art practice the present invention. Any person skilled in the art can easily make further improvements and improvements without departing from the spirit and scope of the present invention. Therefore, the present invention is only limited by the content and scope of the claims of the present invention, and it is intended to cover all those included in the appendix Alternatives and equivalents within the spirit and scope of the present invention as defined by the claims.

## Claims

1. The application of pyrroloquinoline quinone in the preparation of medicines for preventing and treating acute altitude sickness.

2. The application of pyrroloquinoline quinone in the preparation of drugs for the prevention and treatment of acute altitude hypoxia injury.
